## Europäisches Patentamt

## European Patent Office

## Office européen des brevets

(11) Publication number: **0 141 642**
**A1**

# EUROPEAN PATENT APPLICATION

(21) Application number: **84307472.5**

(22) Date of filing: **30.10.84**

(51) Int. Cl.⁴: **A 23 C 21/02, C 12 P 7/52**

(30) Priority: **02.11.83 US 548170**
**02.11.83 US 548175**
**15.10.84 US 659571**

(43) Date of publication of application: **15.05.85**
**Bulletin 85/20**

(84) Designated Contracting States: **CH DE FR GB LI SE**

(71) Applicant: **STAUFFER CHEMICAL COMPANY, Westport Connecticut 06881 (US)**

(72) Inventor: **Bodie, Elizabeth Ann, 904 Kearney, El Cerrito California 94530 (US)**
Inventor: **Schwartz, Robert David, 3042 Santa Maria Court, Concord California (US)**
Inventor: **Anderson, Thomas Marshall, 22 Emery Bay Drive, Emeryville California 94608 (US)**

(74) Representative: **Smith, Sydney et al, Elkington and Fife High Holborn House 52/54 High Holborn, London WC1V 6SH (GB)**

(54) **Improved fermentation process.**

(57) An improved process for manufacture of fermented whey having a high concentration of propionic acid is disclosed in which the whey medium having a solids content ranging from about 0.5% to about 18% by weight is subjected to a sterilization step of heating to 121 °C psig for 15 minutes or subjected to a sterilization step of direct steam injection, for a period of about 4 to about 20 seconds. The sterilized whey medium is then inoculated with an unacclimated or acclimated mixed culture of a propionic acidproducing bacterium and a lactic acid-producing bacterium and incubated for a sufficient period of time to maximize the fermentation.

ACTORUM AG

PR-6592/6995A, B & C

## IMPROVED FERMENTATION PROCESS

### Background of the Invention

Whey, a naturally occuring substance, is the serum or watery part of milk which is separated from the curds in the process of making cheese. Dried whey is comprised of about 13% protein and 73% lactose, and the balance inorganic salts. One of the uses of whey in commerce is to ferment it and incorporate it into bakery products, where it acts as a mycostatic agent, thus enabling the products to have substantially longer shelve lives than would otherwise be possible. It has been determined that the active mycostatic agent in whey is propionic acid which is produced as a consequence of the fermentation process.

Suggested bacteria for fermentation, as disclosed in U.S. Patents, 1,910,130 and 1,898,320, include Bacterium acidi-propionici. In producing fermented whey, it is desirable to sterilize the whey and to ferment it with a known non-pathogenic culture in order to keep out pathogens, and control the product quality and specifications. One of the steps in the process of fermentation is the sterilization of the whey substrate, which must be done without adversely affecting the subsequent fermentation. Sterilization is necessary in order to kill any residual organisms in the whey prior to introducing the organism for fermentation.

The bacterium organism of choice for the fermentation is a strain of Propionibacterium shermanii, although other propionic acid producing bacteria can be used. In the normal prior art process, the whey is sterilized by autoclaving at 121°C, at 15 psig, for 15 minutes. The concentration of the whey component being sterilized is about 2% by weight. It has been found that when whey having a solids concentration of more than about 2% is sterilized by autoclave, successful subsequent fermentation with the Propionibacterium shermanii is somehow prevented. It is desirable to raise the solids concentration of the whey being sterilized so as to provide a high solids broth that increases the concentration of propionic acid produced in the fermentation process, and also to produce a concentration that is easier to process downstream, as by air drying.

Lactobacilli and Propionibacteria are often grown together. In fermented dairy products, such as Swiss cheese, the action of both microorganisms is necessary to obtain the desirable characteristics of the final product (4). In silage fermentations, lactic acid bacteria are needed for rapid pH reduction and propionic acid inhibits the growth of undesirable yeast (12). In early propionic acid fermentation, lactic acid bacteria were used as "accelerator" organisms as disclosed in U.S. Patent No. 1,895,329: Sherman, J.M. 1932. Propionic acid fermentation by the use of mixed strains of propionic bacteria. U.S. Patent 1,865,146 and Sherman, J.M. and R.H. Shaw, 1923. The propionic acid fermentation of lactose. J. Biol. Chem., 56:695-708.

## Description of the Drawings

Figure I shows a graph of a fermentation of *Propionibacterium shermanii* ATCC 39393 and *Lactobacillus casei* ATCC 39392 in a medium containing 7% Teklac (whey) and 1.0% yeast extract.

Figure Ia shows a graph of a fermentation of *Propionibacterium shermanii* ATCC 39393 in a medium containing APV sterilized 12% Teklac (whey) and 1% yeast extract.

Figure II shows a graph of a fermentation of *Propionibacterium shermanii* ATCC 39393 and *Lactobacillus casei* ATCC 39392 in a medium containing 12% Teklac (whey) and 1.0% yeast extract.

Figure IIa shows a graph of a fermentation of *Propionibacterium shermanii* ATCC 39393 and *Lactobacillus casei* ATCC 39392 in a medium containing APV sterilized 12% Teklac (whey) and 1% yeast extract.

Figure IIb shows a graph of a fermentation of acclimiated *Propionibacterium shermanii* ATCC 39393 and *Lactobacillus casei* ATCC 39392 in a medium contianing APV sterilized 12% Teklac (whey) and 1% yeast extract.

Figure III shows a graph of a fermentation of *Propionibacterium shermanii* ATCC 39393 and *Lactobacillus casei* ATCC 39392 in a medium containing APV sterilized 18% Teklac (whey) and 1.5% yeast extract.

## Description of the Invention

Sterilizing whey medium at a whey concentration greater than 2% by autoclaving at 121°C and 15 psig for at least 15 minutes results in a medium that can not be fermented by P. shermanii. This inhibition most likely results from the harse and destructive method of sterilization. When whey is autoclaved a browning reaction occurs, where the free amino groups of the protein interact with the aldehyde group of lactose, resulting in loss of nutritional value. The lactose may also undergo a rearrangement to lactulose and become unavailable to P. shermanii. Autoclaving may also produce toxic substances and metabolic inhibitors. See Gordon, W.G. and E.B. Kalan. 1974. Proteins of milk. p. 102-103. In B.H. Webb, A.H. Johnson, and J.A. Alford (eds.). Fundamentals of Dairy Chemistry, 2nd ed. The AVI Publishing Company, Westport, Connecticut. Huthanen, C.N., F.W. Parrish, and K.B. Hicks (1980). Inhibition of bacteria by lactulose preparations. Appl. Environ. Microbiol. 40:171-173. Thayanithy, K., G. Harding and D.A. Wase. 1982. Rearrangement of lactose on sterilization. Bio. Technol. Lett. 4:423-444.

The inhibition was overcome by adding L. casei to P. shermanii (mixed culture) in whey medium sterilized by autoclaving as described above and propionic acid was produced. The yield was 1.3-2.2% in 7% whey solids and 3.0% in 12% whey solids in about 88 hours.

Sterilizing whey solutions having a solids content from about 0.5 to about 18% by subjecting said whey solution to a direct steam injection while being passed through a transport mechanism wherein the steam has a temperature high enough to produce a medium temperature for the solution of at least 140°C, for a period of time ranging from about 4 to about 20 seconds (UHT), a sterile whey solution of high solids content is produced which can be subsequently fermented by a pure culture of P. shermanii or other propionic acid-producing bacteria thereby producing high concentrations of propionic acid. The yield was 1.6-2.2% propionic acid within about 70 hours on 12% whey solids.

We have further discovered that by combining UHT sterilization and mixed culture fermentation using P. shermanii and L. casei high solids whey medium containing up to 18% whey solids can be fermented to even higher concentrations of propionic acid. Propionic acid concentrations as great as 3.2% have been achieved in less than 50 hours, using 12% whey solids. Further, 6.5% propionic acid was produced in 18% whey solids within about 100 hours.

We have further discovered that by prior acclimation of the mixed culture to the whey medium at least 4.5% propionic acid is produced within about 70 hours using 12% whey solids, compared to about 3% propionic acid using unacclimated mixed cultures. Higher concentrations of Teklac will also produce comparable increases in propionic acid production when acclimated cultures are used.

Mixed culture fermentation of a sterilized whey broth comprising unhydrolyzed whey (acid or sweet), and optionally yeast extract results in propionic acid formation and functionalization of the whey so that the whey product can be utilized as a food ingredient. This anaerobic fermentation can be carried out preferably in a pH range of 5.5 to 8.5, preferably with the pH maintained in a range from about 6.0 to about 7.0. The fermentation can be carried out at a temperature from about 20 to 35°C, preferably carried out at a temperature from about 25 to about 30°C. Typical composition of Teklac (sweet dairy whey) is as follows:

CHEMICAL AND PHYSICAL SPECIFICATIONS

Ingredient
Listing:    Whey

<u>Typical Proximate Analysis</u>

| | |
|---|---|
| Protein (N x 6.38)% | 12.7 |
| Fat % | 1.1 (1.25% Maximum) |
| Moisture % | 4.5 (5.0% Maximum) |
| Ash % | 8.0 |
| Lactose % | 71.3 |
| Calories, Cal/100g | 350.0 |

0141642

Typical Vitamin & Mineral Analysis

| | |
|---|---|
| Vitamin A I.U./100g | Nil |
| Vitamin C mg/100g | Nil |
| Thiamin mg/100g | 0.40 |
| Riboflavin mg/100g | 1.76 |
| Niacin mg/100g | 1.00 |
| Calcium % | 0.71 |
| Iron % | Nil |
| Vitamin $B_{12}$ ug/100g | 2.12 |
| Phosphorus % | 0.69 |
| Pantothenic Acid mg/100g | 4.09 |

Microbiological Standards

| | |
|---|---|
| Standard Plate Count | 10,000/g (Maximum) |
| Coliforms | 9/g (Maximum) |
| E. coli | Negative |
| Salmonella | Negative |

The nutritional values listed above are within 80% of the value declared in compliance with Federal Nutritional Regulations 21 CFR §1.17(4)(ii).

| | Typical Range | Limit |
|---|---|---|
| Solubility Index | 0.1 – 0.5 ml | 1.25 ml Max. |
| Acidity | 0.10 – 0.14% | 0.16 Max. |
| Alkalinity of Ash | 175 – 200 ml | 225 ml Max. |
| Scorched Particles | 7.5 mg | 15.0 mg Max. |
| Particle size (Through 40 Mesh) | 99 – 100% | 98% Min. |

Concentration of whey solids can range from >2% to about 18.0%, preferably from about 6% to about 12%, and the concentration of added yeast extract in the fermentation broth can range from about 0.5% to about 4.0%, preferably from about 0.5% to about 1.5%. Using autoclaved whey and mixed culture, propionic acid concentrations of from about 1.0% to about 3.5% are usually reached within 96 hours. Using UHT sterilized whey and mixed culture, propionic acid concentrations of about 3.0% are usually reached within 55 hours using 12% whey solids; propionic acid concentrations of about 6.5% are usually reached within 100 hours using 18% whey solids. Using UHT sterilized whey and an acclimated mixed culture, propionic acid concentrations of about 4.5% are usually reached within about 72 hours using 12% whey solids. All of the above weight percents are in weight per volume.

## EXAMPLES

### Microorganism

Lactobacillus casei ATCC 39392 was isolated from Kraft Swiss cheese. Twenty grams (g) of Kraft natural Swiss cheese were homogenized in 180 milliliters (ml) cold sterile saline for 3 minutes in a Waring blender. The homogenate was plated on sodium lactate agar (10 g trypticase, 10 g yeast extract, 10 g sodium lactate, 0.25 g $K_2HPO_4$, 20 g agar, one liter (l) deionized water) at various dilutions, and incubated for five days, anaerobically, at 30°C. A clonal isolate was subsequently identified as L. casei and its authenticity confirmed by the American Type Culture Collection (Washington, D.C.).

Propionibacterium freudenreichii ss. shermanii ATCC 39393 was a single colony isolate obtained from a PS-1 Dry-Vac specialty culture (Chris Hansen's Laboratory, Milwaukee, WI).

### Whey Medium Preparation, Shake Flask, and Fermentor Operation

The whey media contained 2-12% Teklac and 1% yeast extract. (Teklac is a spray dried sweet dairy whey obtained from Foremost Food Co., San Francisco, CA.) The pH of the medium was adjusted to 7.0 with 50% NaOH before autoclaving. For shake flask reactions 500 ml screw-capped flasks (Bellco, Vineland, NJ) containing 250 ml of whey medium and 2% $CaCO_3$ were used. Sterilization was for 20 minutes, 121°C, 15 psig steam. The shake flasks were incubated at 30°C, 160 rpm on a NBS G-25 shaker (New Brunswick Scientific Co., Edison, NJ). For fermentors, New Brunswick Scientific Co. (Edison, NJ) 7.5 liter Magnaferm fermentors or 2 liter Bioflo fermentors, containing 3 liters and 1.2 liters medium, respectively, were used. The fermentors were filled with about one liter water and sterilized by autoclaving for 50 minutes, 121°C, 15 psig steam. Aseptically, the water was removed and sterilized whey medium pumped in from a carboy. The fermentation conditions were 30°C, 120-160 rpm, no gas sparged, pH controlled at 7.0 with NaOH.

The fermentors were used in either a batch fermentation mode or a "drawn-and-fill" fermentation mode. In a batch mode, medium is pumped into the fermentor which is then inoculated and incubated for several days. A draw and fill mode is used to conduct serial fermentations in the same vessel and to acclimate the cultures through several stages. The broth from a completed stage was removed, except for a small amount to be used as inoculum for the next stage, and fresh medium added on top of the retained inoculum. The first and second stages were incubated for about 24 hours each. The third and final stage was incubated for 72 hours. Inoculation was at about 10% v/v for stages two and three.

## Ultra High Temperature (UHT) Sterilization

An APV heat exchanger (APV CO., Inc. Tonawanda, N.Y.) was modified to use direct steam injection by adding a pick heater. The medium was pumped from the feed tank to a plate heat exchanger where it was preheated to 94°C. After leaving the preheater, the medium was heated in the pick heater, by direct contact with steam, to the sterilization temperature of 146°C. Sterilization took place in a holding coil between the pick heater and the cooling section. The sterilization time, 15-20 seconds, depended on the medium flow rate which was controlled. The hot medium was cooled in a plate heat exchanger, then transferred through a sterile manifold system into sterile carboys for storage. It was later transferred asceptically to fermentors using a peristaltic pump.

## Culture Storage

P. shermanii was stored in sodium lactate stabs (10 g trypticase, 10 g yeast extract, 10 g Na lactate, 0.25 g $K_2HO_4$, 12 g agar, 1 liter deionized water, 5 ml/18 mm x 150 mm screw-capped tube). Stabs were inoculated, incubated at 30°C for 72 hours and stored at 4°C for up to 6 months. A stab was revived by overlaying with 5 ml Hansen's glucose broth (HGB) (10 g trypticase, 5 g yeast extract, 5 g glucose, 1 liter deionized water) and incubating for 48 hours at 30°C.

L. casei was stored in MRS stabs [Lactobacilli MRS broth (Difco) and 2% agar]. Stabs were inoculated, incubated at 30°C for 48 hours and stored at 4°C for up to two months. A stab was revived by overlaying with 5 ml MRS broth and incubating for 48 hours at 30°C.

## Preparation of Inocula

A shake flask containing 2% Teklac medium was inoculated with either 5 ml HGB from one revived stab (P. shermanii) or 5 ml MRS from one revived stab (L. casei). The flasks were incubated for 48 hours. These cultures were then used to inoculate mixed culture shake flasks at 2.5% v/v per culture. Pure culture flasks received a 5% v/v inoculum and were used as controls. Mixed culture reactions were inoculated at 2.5% or 8% v/v for P. shermanii and 2.5% or 8% v/v for L. casei, unless otherwise indicated.

## Assays

D- and L-lactic acids, and lactose were estimated enzymatically using kits purchased from Boehringer-Mannheim (Indianapolis, IN). Propionic acid (HPr) and acetic acid (HAc) were assayed by gas chromatography. A Packard Model 427 with a 6f glass column (I.D. = 2 mm, O.D. = 6 mm) packed with 10% AT-1000 on Chromasorb W (All Tech Associates) and a flame ionization detector were used. The column temperature was 140°C, isothermal; injector and detector temperatures were 250°C. The carrier gas was nitrogen with a flow rate of 18 ml per minute. Samples were prepared by centrifuging 5 ml broth (to remove cells and other suspended solids) and acidifying the supernatant with 1% $H_3PO_4$ to a pH of 2.0. Three microliters of the acidified supernatant were injected using a 10 ul Hamilton N701 syringe. Results were calculated from a standard curve.

## Determination of Viable Cell Number (CFU) in Mixed Culture

P. shermanii and L. casei together in mixed culture were assayed simultaneously on sodium lactate gar (10 g trypicase, 10 yeast extract, 10 g sodium lactate, 0.25 g $K_2HPO_4$, 20 g agar, 1 liter deionized water Spread plates were incubated anaerobically (Gaspak 100, BBL 60627) at 30°C for four days. Under these conditions, P. shermanii grew as a smaller, tan-colored colony; L. casei grew as a larger, milk-white colony.

## EXAMPLE I

### Shake Flask Reactions

At a Teklac concentration of 2% pure cultures of P. shermanii grew to $1.0 \times 10^{10}$ colony forming units/ml (CFU/ml), produced about 0.6%

propionic acid and consumed all the lactose (about 14 g/l). At Teklac concentrations higher than 2%, no growth occurred, a negligible amount (less than 0.05%) of propionic acid was produced, and lactose was not metabolized. Pure cultures of L. casei grew to about 1 x $10^9$ CFU/ml and converted 80-85% of the lactose to lactic acid at all Teklac concentrations examined. For example, in 7% Teklac (about 49 g lactose/l), L. casei produced 40-42 g lactic acid/l and consumed all the lactose.

Typical mixed culture shake flask data for various Teklac concentrations are summarized in Table I. In 72 hours, at Teklac concentrations of 5, 6 and 7%, about 1.3-1.6% propionic acid and 0.5% acetic acid were produced and most of the lactose was consumed. Lactic acid concentrations remained low throughout the fermentation.

### TABLE I

Mixed culture fermentation using P. shermanii PS-109
and L. casei TA-101 growing in shake flasks.

| Teklac %[a] | Lactose, g/l at $t_0$ | 48 hours g/l | | | | | 72 hours g/lg | | | | |
| --- | --- | --- | --- | --- | --- | --- | --- | --- | --- | --- | --- |
| | | pH | Lactose | Lactic acid | HAc[b] | HPr[b] | pH | Lactose | Lactic acid | HAc[b] | HPr[b] |
| 5 | 38 | 5.30 | 4 | 2.2 | 3.8 | 10.3 | 5.84 | <0.5 | <0.5 | 5.3 | 12.5 |
| 6 | 44 | 5.26 | 17 | 2.8 | 3.7 | 9.1 | 5.60 | <0.5 | 4.4 | 5.2 | 16.0 |
| 7 | 47 | 5.26 | 22 | 3.2 | 3.9 | 9.5 | 5.44 | 2.7 | 7.8 | 5.3 | 15.5 |

a) The media also contained 1% yeast extract and 2% $CaCO_3$. Initial pH 6.90. Inocula ratio   1:8, ATCC 39392 to ATCC 39393.

b) HAc, Acetic acid; HPr, Propionic acid.

### EXAMPLE II

#### Fermentor Reactions

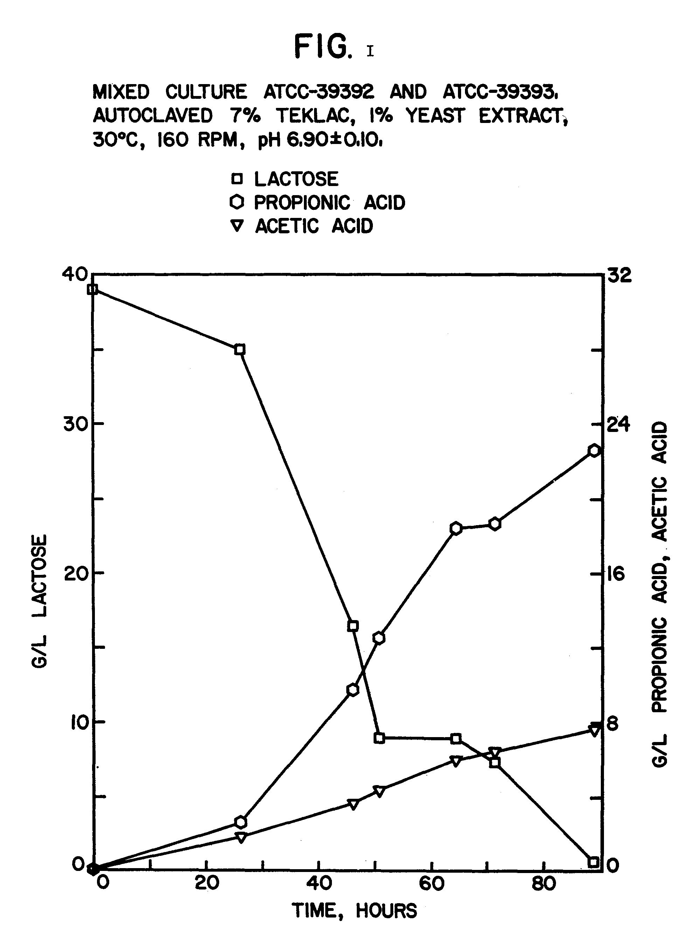

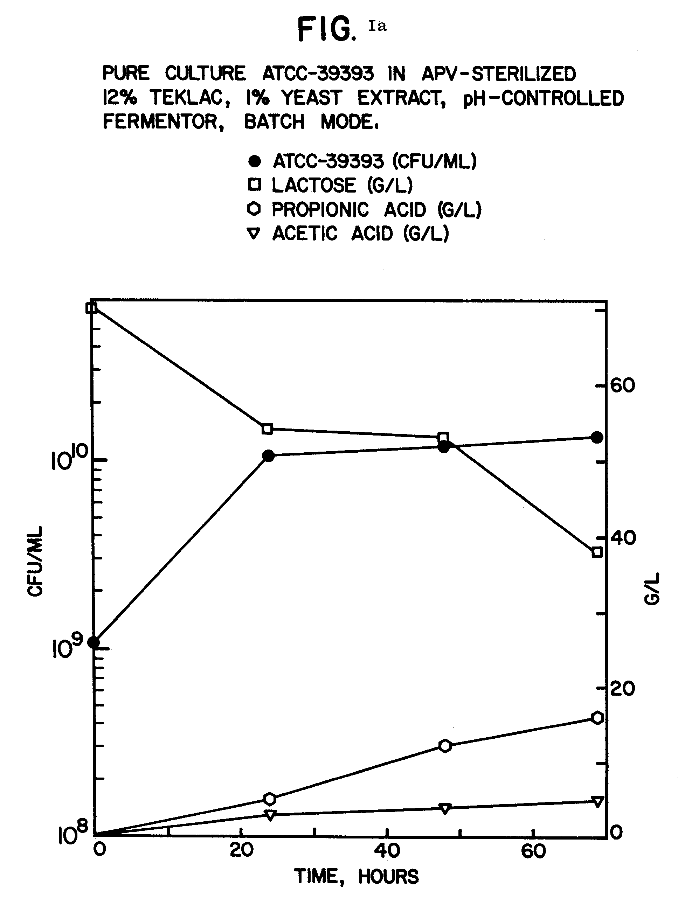

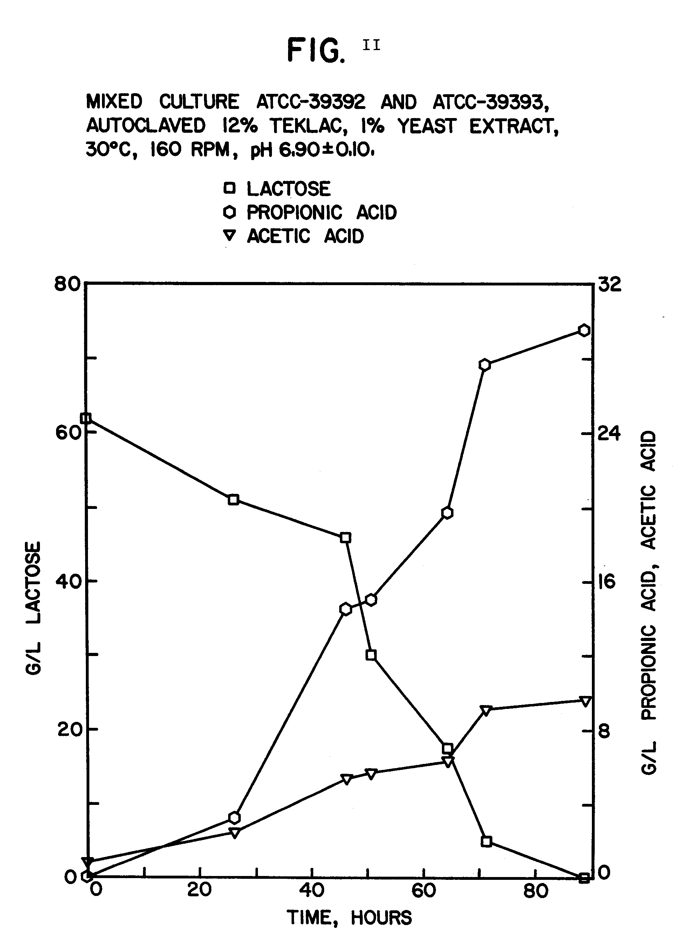

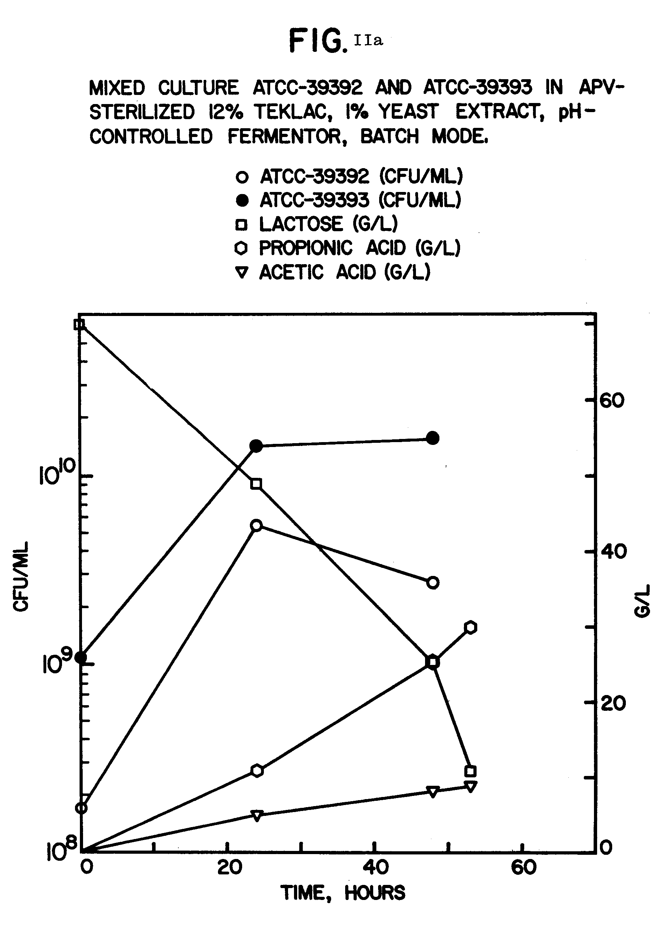

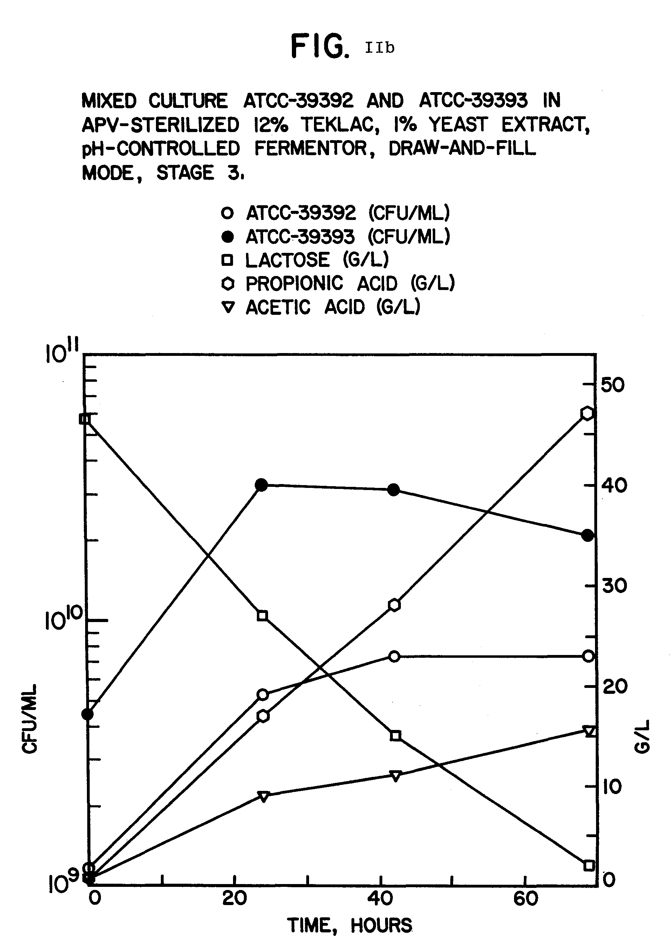

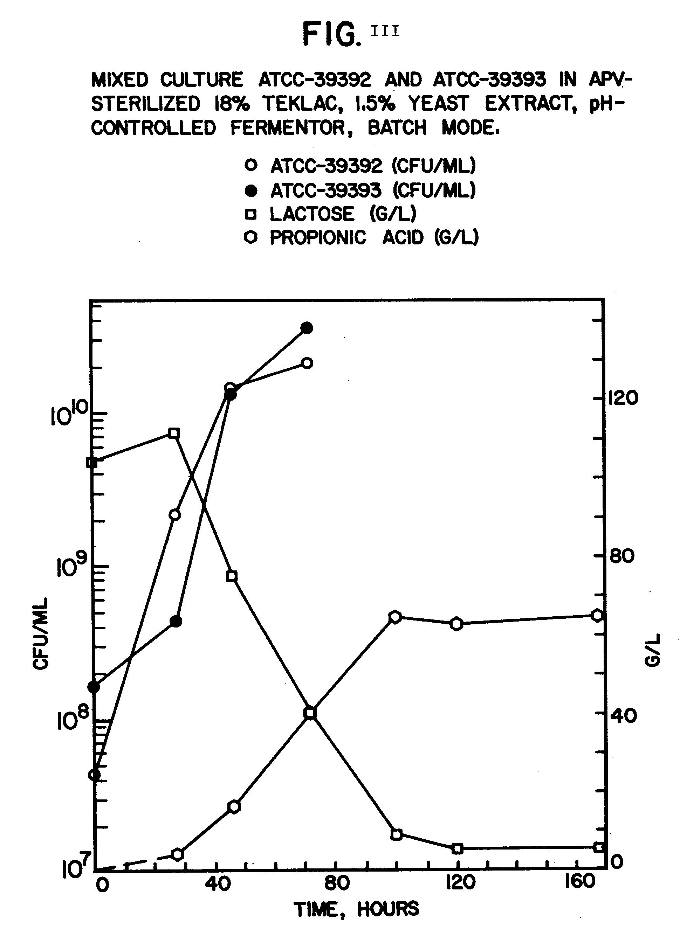

Results from pH controlled mixed culture fermentor reactions using 7% and 12% autoclaved Teklac are shown in Figures 1 and 2, respectively. Within 88 hours, in 7% Teklac, about 2.2% propionic acid and 0.8% acetic acid were produced. In 12% Teklac, bout 3.0% propionic and 1% acetic acid were produced. In both reactions all the lactose was consumed and lactic acid did not accumulate.

## EXAMPLE III

Batch fermentations using pure culture P. shermanii ATCC 39393 resulted in 1.6-2.2% propionic acid produced within 70 hours. More than 50% of the lactose was not used. Figure Ia shows a typical reaction. In mixed culture, L. casei ATCC 39392 and P. shermanii ATCC 39393 produced about 3.2% propionic acid and most of the lactose was consumed within 55 hours. (See Figure IIa.)

## EXAMPLE IV

When the Teklac concentration was increased to 18% (with 1.5% Kat yeast extract), the fermentor operated in the batch mode and inoculated at 1% v/v for each culture about 6.5% propionic acid was produced in about 100 hours. All the lactose was consumed (see Figure III).

## EXAMPLE V

Batch fermentations using unacclimated mixed cultures of P. shermanii ATCC 39393 and L. casei ATCC 39392 resulted in about 3.0% propionic acid produced within 70 hours. Figure IIa shows a typical reaction. In mixed culture, L. casei ATCC 39392 and P. shermanii ATCC 39393 produced about 3.2% propionic acid and most of the lactose was consumed.

## EXAMPLE VI

P. shermanii ATCC 39393 and L. casei ATCC 39392 grown together (acclimated) through three stages in a draw and fill mode, produced about 4.5% propionic acid in 70 hours. All the lactose was consumed (Figure IIb).

- 11 -                                    0141642

CLAIMS:

1.    A process for functionalizing whey containing up to
2% whey solids by weight by fermenting the whey with a
propionic acid-producing organism to produce propionic
acid wherein the whey has been sterilized by autoclaving
at 121$^{o}$C at 15 psig for 15 minutes characterised in that
     (a) the whey solids are increased to from above
     about 2% to about 18% by weight;
     (b) a fermentation broth with the increased whey
     solids and yeast extract is produced;
     (c) the fermentation broth is fermented with a
     mixed culture comprising a propionic acid-producing
     bacterium and lactic acid-producing bacterium to
     produce propionic acid concentrations greater than
     1% by weight.

2.    A process for functionalizing whey containing from
about 0.5% to about 18% by weight whey solids by fermenting
the whey with a propionic acid-producing organism to
produce propionic acid wherein the whey has been sterilized
by direct steam injection that results in a whey medium
temperature of at least 140$^{o}$C held for from about 4 to
about 20 seconds, characterised in that
     (a) a fermentation broth is formed with the whey
     solids and yeast extract; and
     (b) the fermentation broth is fermented with a
     mixed culture comprising a propionic acid-producing
     bacterium and lactic acid-producing bacterium to
     produce propionic acid concentrations greater than
     1% by weight.

3.    A process as claimed in claim 1 or claim 2 characterised in that the propionic acid bacterium is _Propionibacterium_ _shermanii_ and the lactic acid bacterium is
_Lactobacillus_ _casei_.

4.    A process as claimed in claim 1 or claim 2
characterised in that the propionic acid bacterium is
_Propionibacterium_ _shermanii_ ATCC 39393 and the lactic
acid bacterium is _Lactobacillus_ _casei_ ATCC 39392.

5.    A process as claimed in any of claims 1 to 4 characterised in that the concentration of the yeast extract is from about 0.5 to about 4.0% by weight.

6.    A process as claimed in any of claims 1 to 6 characterised in that the concentration of the whey solids is from about 6.0 to about 12.0% by weight.

7.    A process as claimed in any of claims 1 to 6 characterised in that the propionic acid-producing organism and the lactic acid-producing organism are acclimated to each other and the culture medium by growing the two cultures together for several serial passages whereby production of propionic acid is enhanced by at least 25% compared to production of non-acclimated cultures.

116

0141642

## FIG. I

MIXED CULTURE ATCC-39392 AND ATCC-39393,
AUTOCLAVED 7% TEKLAC, 1% YEAST EXTRACT,
30°C, 160 RPM, pH 6.90±0.10,

□ LACTOSE
○ PROPIONIC ACID
▽ ACETIC ACID

FIG. Ia

PURE CULTURE ATCC-39393 IN APV-STERILIZED
12% TEKLAC, 1% YEAST EXTRACT, pH-CONTROLLED
FERMENTOR, BATCH MODE.

● ATCC-39393 (CFU/ML)
□ LACTOSE (G/L)
○ PROPIONIC ACID (G/L)
▽ ACETIC ACID (G/L)

3/6

0141642

## FIG. II

MIXED CULTURE ATCC-39392 AND ATCC-39393,
AUTOCLAVED 12% TEKLAC, 1% YEAST EXTRACT,
30°C, 160 RPM, pH 6.90±0.10.

□ LACTOSE
○ PROPIONIC ACID
▽ ACETIC ACID

416

0141642

# FIG. IIa

MIXED CULTURE ATCC-39392 AND ATCC-39393 IN APV-
STERILIZED 12% TEKLAC, 1% YEAST EXTRACT, pH-
CONTROLLED FERMENTOR, BATCH MODE.

○ ATCC-39392 (CFU/ML)
● ATCC-39393 (CFU/ML)
□ LACTOSE (G/L)
⬡ PROPIONIC ACID (G/L)
▽ ACETIC ACID (G/L)

6/6

0141642

FIG. IIb

MIXED CULTURE ATCC-39392 AND ATCC-39393 IN
APV-STERILIZED 12% TEKLAC, 1% YEAST EXTRACT,
pH-CONTROLLED FERMENTOR, DRAW-AND-FILL
MODE, STAGE 3.

○ ATCC-39392 (CFU/ML)
● ATCC-39393 (CFU/ML)
□ LACTOSE (G/L)
⬠ PROPIONIC ACID (G/L)
▽ ACETIC ACID (G/L)

616

0141642

# FIG. III

MIXED CULTURE ATCC-39392 AND ATCC-39393 IN APV-
STERILIZED 18% TEKLAC, 1.5% YEAST EXTRACT, pH-
CONTROLLED FERMENTOR, BATCH MODE.

○ ATCC-39392 (CFU/ML)
● ATCC-39393 (CFU/ML)
□ LACTOSE (G/L)
○ PROPIONIC ACID (G/L)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-2 465 905  (R. MEADE)  <br><br>* claim 1; examples 1-3 * | 1,2,5-7 | A 23 C   21/02 <br> C 12 P    7/52 |
| P,Y | EP-A-0 096 477  (STAUFFER CHEMICAL CO.) <br> * claims  1-8; page 1, line 25 - page 2, line 6; examples 1,2 * | 1,2,5-7 | |
| A | US-A-2 020 251  (H. STILES) <br> * claim  1;  column  2, line 43; example IV * | 3,4 | |
| A | US-A-1 910 130  (J. SHERMAN) <br> * claim 1; column 1, lines 30-44 * | 1 | |

TECHNICAL FIELDS SEARCHED (Int. Cl.4)

A 23 C
C 12 P

The present search report has been drawn up for all claims

| Place of search | Date of completion of the search | Examiner |
|---|---|---|
| THE HAGUE | 06-02-1985 | DESMEDT G.R.A. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503. 03.82